# EUROPEAN PATENT SPECIFICATION

(11) **EP 4 536 173 B1**
(45) Date of publication and mention of the grant of the patent: **10.09.2025**
(21) Application number: 23731206.1
(22) Date of filing: 06.06.2023
(51) Int. Cl.: A61K 8/26, A61K 8/894, A61K 8/92, A61Q 15/00

(54) **ANTIPERSPIRANT COMPOSITION**
SCHWEISSHEMMENDE ZUSAMMENSETZUNG
COMPOSITION ANTITRANSPIRANTE

(30) Priority: 08.06.2022 EP 22177918; 08.06.2022 EP 22177924
(43) Date of publication of application: 16.04.2025
(73) Proprietor: Unilever IP Holdings B.V., 3013 AL Rotterdam (NL); Unilever Global IP Limited, Wirral, Merseyside CH62 4ZD (GB)
(72) Inventor: DAS, Somnath, 6708 WH Wageningen (NL); NYALAM, Praveen, 6708 WH Wageningen (NL)
(74) Representative: Whaley, Christopher
(86) International application number: PCT/EP2023/065055
(87) International publication number: WO 2023/237517

(56) References cited:
- WO-A1-2011/050044
- WO-A1-2021/028180
- DE-A1- 102016 205 698
- US-A1- 2012 121 525
- US-A1- 2019 105 239
- US-B2- 10 172 773

## Description

### Field of the Invention

The present invention relates to an antiperspirant composition.

### Background of the Invention

Many consumers complain about undesirable stains and marks in the underarm area in clothing with repeated use of antiperspirants. These are frequently noticeable as yellowish stains which may also tend to become incrusted. These stains are produced due to a complex interaction between antiperspirant composition, skin fat, sweat and are often difficult to remove with conventional washing methods. These stubborn stains are often difficult to remove. Marks are produced by the transfer of antiperspirant particles from the skin to the fabric and also due to residue build up of the antiperspirant ingredients on multiple wash wear.

Antiperspirant actives are used widely in antiperspirant compositions. These actives are typically astringent metal salts such as aluminium or zirconium salts e.g., aluminium chlorohydrates or sesquichlorohydrates. These actives have the effect of reducing perspiration, particularly when applied onto the underarm regions of the human body viz. the axilla. One cause for the stains is the aluminum salts which act as antiperspirant active substance in most antiperspirant compositions. In combination with the sweat the antiperspirant actives form a yellow stain. Further, in a normal machine-washing cycle this yellow stain is accentuated by washing, and the heat (electric dryer, ironing or pressing) can further accentuate the intensity of the coloring.

Increasingly, current antiperspirant compositions also include natural oils for delivering skin care benefits. The natural oil includes unsaturated oils such as sunflower seed oil. The present inventors have found that when antiperspirant compositions having metal based antiperspirant active and an unsaturated oil are applied on the underarm region, the fabric which comes in contact with the composition tends to get stained on repeated use (where the fabric may be washed, rinsed and dried between each use). It was also found that these stains are stubborn and are even more prominent when the composition includes an unsaturated natural oil, such as sunflower seed oil.

While one could reduce the level of yellowing by lowering the level of the antiperspirant active and the natural oils, but any such reduction will also reduce the efficacy and the antiperspirant composition would not be effective. There is a consumer desire to reduce or eliminate yellowing of clothing while still maintaining the effectiveness of the antiperspirant composition and which provides desired skin care benefits.

Non-ionic surfactants have been used in the antiperspirant compositions for various purpose. Surfactants are generally included to emulsify the oily components.

One such document is US 2010/0260698 A1 (Henkel) which discloses an anhydrous antiperspirant composition providing improved antiperspirant performance by allowing better release of antiperspirant active. The antiperspirant composition includes at least one organosiloxane-oxyalkylene copolymer having an HLB value in the range from 8 to 20.

DE 10 2016 205698 A1 (Henkel, 2017) discloses an anhydrous antiperspirant composition for reducing staining on fabrics. The composition includes antiperspirant actives non-ionic emulsifiers having an HLB value of less than 11 adsorbed on silica particles, and a natural oil as hydrophobic carrier.

It is desirable to provide cosmetic antiperspirant composition that has a reduced soiling of clothing and increases the ability to subsequently wash out the soiling.

It is thus an object of the present invention to provide an anhydrous antiperspirant composition comprising a metal based antiperspirant active and natural oil that minimizes the problem of staining or yellowing of fabrics on repeated use-wash-rinse-dry cycles of the fabric when worn by individuals using such antiperspirant composition.

In addition, the composition must not have any instabilities and should be easy to formulate.

It is yet another object of the present invention to provide cosmetic anhydrous antiperspirant composition that minimizes the problem of staining or yellowing of fabrics and increasing the washability of stains while maintaining good skin tolerance.

### Summary of the Invention

The present inventors have surprisingly found that when specific silicone nonionic surfactant having a HLB value less than 11 is included in an antiperspirant composition comprising a metal based antiperspirant active and an unsaturated oil, then the yellow stain is easily removed when the fabric is subsequently washed. The inventive anhydrous antiperspirant composition provides for reducing or eliminating the yellowing of clothing while still maintaining the effectiveness of the antiperspirant composition and providing good skin tolerance.

More particularly, it was found that the silicone nonionic surfactant also ensures complete removal of the more pronounced yellow stain formed when the composition has an unsaturated oil, particularly sunflower seed oil. According to the invention it has now surprisingly shown that the nonionic silicone surfactant having a HLB value less than 11, improve the ability to wash out stains.

It is further found that the anhydrous antiperspirant composition according to the present invention having specific silicone nonionic surfactant having an HLB value of less than 11 when included in an antiperspirant composition comprising a metal based antiperspirant active and an unsaturated oil enables to minimize or reduce the white spots or marks formed on the black fabrics which are consumer perceivable. It helps in reducing particulate build up on the fabrics on repeated wash wear, thus reducing the white marks on black fabrics.

According to a first aspect of the present invention disclosed is an antiperspirant composition comprising:
i) a metal based antiperspirant active;
ii) an unsaturated oil having an iodine value of at least 100;
iii) 1 wt.% to 20 wt.% non-ionic silicone surfactant having an HLB value of less than 11;
iv) less than 10 wt.% water.

By "An Antiperspirant Composition" as used herein, is meant to include a composition for topical application to the skin of mammals, especially humans. Such a composition is preferably of the leave-on type. By a leave-on composition is meant a composition that is applied to the desired skin surface and left on for a period of time (say from one minute to 24 hours) after which it may be wiped or rinsed off with water, usually during the regular course of personal washing. The composition may also be formulated into a product which is applied to a human body for improving the appearance, cleansing, odor control or general aesthetics. The composition of the present invention can be in the form of a liquid, lotion, cream, foam, scrub, gel or stick form. Alternatively they may be delivered through a roll-on device or by using a propellant containing aerosol can. It is especially useful for delivering low pH compositions to the axilla of an individual for anti-perspirancy benefits. "Skin" as used herein is meant to include skin on any part of the body (e.g., neck, chest, back, arms, underarms, hands, legs, buttocks, and scalp) especially the underarms.

### Detailed Description of the Invention

According to a first aspect of the present invention disclosed is an antiperspirant composition having a metal based antiperspirant active, 1 wt.% to 20 wt.% silicone non-ionic surfactant having an HLB value of less than 11, an unsaturated oil having an iodine value of at least 100, and less than 10 wt.% water.

### Metal based antiperspirant active

The anti-perspirant composition according to the first aspect of the present invention includes a metal based antiperspirant active.

The metal based antiperspirant active may be selected from an aluminium, zirconium or mixed aluminium/zirconium salts, preferably, aluminium chlorohydrate, aluminum-zirconium tetrachlorohydrex glycine complex, aluminum-zirconium octachlorohydrex glycine complex, aluminum-zirconium pentachlorohydrate, aluminum sesquichlorohydrate or mixtures thereof. Antiperspirant actives for use herein are selected from aluminium, zirconium and mixed aluminium/zirconium salts, including both inorganic salts, salts with organic anions and complexes. Particularly preferred astringent salts are halohydrate salts, and especially chlorohydrate salts, optionally activated. Preferably for aerosol compositions, the antiperspirant active is preferably free from zirconium.

Aluminium halohydrates are usually defined by the general formula Al₂(OH)ₓQ_{y}.wH₂O in which Q represents chlorine, bromine or iodine, x is variable from 2 to 5 and x + y = 6 while _{W}H₂O represents a variable amount of hydration. Especially effective aluminium halohydrate salts, known as activated aluminium chlorohydrates, are described in EPA-6739 (Unilever NV et al).

The term aluminium chlorohydrate herein encompasses materials with specified figures for x and y, such as aluminium sesquichlorohydrate and materials in which the chlorohydrate is present as a complex. It will be recognized that alternative names are sometimes used to indicate the presence of hydroxyl substitution, including aluminium hydroxychloride, aluminium oxychloride or basic aluminium chloride.

Zirconium astringent salts for employment herein can usually be represented by the empirical general formula: ZrO(OH)_{2n-nz}B_{z}.wH₂O in which z is a variable in the range of from 0.9 to 2.0 so that the value 2n-nz is zero or positive, n is the valency of B, and B is selected 10 from the group consisting of chloride, other halide, sulphamate, sulphate and mixtures thereof. Possible hydration to a variable extent is represented by wH₂O. Preferably, B represents chloride. Preferably, the variable z lies in the range from 1.5 to 1.87. In practice, such zirconium salts are commonly not employed by themselves, but as a component of a combined aluminium and zirconium-based antiperspirant.

The above aluminium and zirconium salts may have coordinated and/or bound water in various quantities and/or may be present as polymeric species, mixtures or complexes. In particular, zirconium hydroxy salts often represent a range of salts having various amounts of the hydroxy group. Zirconium aluminium chlorohydrate may be particularly preferred.

Antiperspirant complexes based on the above-mentioned astringent aluminium and/or zirconium salts can be employed. The complex often employs a compound with a carboxylate group, and advantageously this is an amino acid. Examples of suitable amino acids include dl-tryptophan, dl-β-phenylalanine, dl-valine, dl-methionine and β alanine, and preferably glycine which has the formula CH₂(NH₂)COOH. Certain of those Al/Zr complexes are commonly called ZAG in the literature. ZAG actives generally contain aluminium, zirconium and chloride with an Al/Zr ratio in a range from 2 to 10, especially 2 to 6, an Al/Cl ratio from 2.1 to 0.9 and a variable amount of glycine. Actives of this preferred type are available from Westwood, from Summit and from Reheis. Alternatively, the complex can be preformed with a polyhydric aliphatic alcohol, such as propylene glycol or glycerol. A complex with a chlorohydrate is commonly referred to as a chlorhydrex.

Mixtures of two or more astringent salts can be employed, but, however, it is particularly preferred to employ astringent salts that are free from zirconium, such as aluminium chlorohydrates and so-called activated aluminium chlorohydrates. As per an especially preferred aspect of the present invention the antiperspirant active is aluminium chlorohydrate, aluminum sesquichlorohydrate or mixtures thereof.

The metal based antiperspirant active is preferably present in an amount ranging from 2 wt.% to 30 wt.% in the antiperspirant composition. Preferably the antiperspirant composition comprises at least 3 wt.%, still preferably at least 5 wt.%, still preferably at least 6 wt.%, most preferably at least 10 wt.% of the metal based antiperspirant active, but typically not more than 25 wt.%, still preferably not more than 20 wt.%, still further preferably not more than 18 wt.%, still more preferably not more than 16 wt.% and most preferably not more than 15 wt.%, of a metal based antiperspirant active based on the weight of the antiperspirant composition.

### Unsaturated oil

The anti-perspirant composition according to the first aspect of the present invention includes an unsaturated oil having an iodine value of at least 100.

As used herein, "iodine value" is the number of grams of iodine that an unsaturated compound or blend will absorb in a given time under arbitrary conditions. A low iodine value implies a high level of saturation, and vice-versa. The iodine value can be determined by the WIJ'S method of the American Oil Chemists Society (A.O.C.S. Cd1-25).

The unsaturated oil are preferably natural oils, preferably a glyceride oil derived from one or more unsaturated fatty acids. In many instances, the oils comprise one or more triglycerides. The fatty acid residues in the oil can comprise, commonly from one to three olefinic unsaturated bonds and often one or two. Whilst in many instances the olefinic bonds adopt the trans configuration, in a number of desirable products the bond or bonds adopt the cis configuration. If two or three olefinic unsaturated bonds are present, they can be conjugated. The fatty acid can also be substituted by an hydroxyl group.

The unsaturated natural oils employable herein desirably comprise one or more triglycerides of oleic acid, linoleic acid, linolenic acid or ricinoleic acid. Various isomers of such acids often have common names, including linolenelaidic acid, trans 7-octadecenoic acid, parinaric acid, pinolenic acid punicic acid, petroselenic acid and stearidonic acid. It is especially desirable to employ glycerides derived from oleic acid, linoleic acid or petroselenic acid, or a mixture containing one or more of them.

Natural unsaturated oils containing one or more of such triglycerides include coriander seed oil for derivatives of petroselinic acid, impatiens balsimina seed oil, parinarium laurinarium kernel fat or sabastiana brasilinensis seed oil for derivatives of cis- parinaric acid, dehydrated castor seed oil, for derivatives of conjugated linoleic acids, borage seed oil and evening primrose oil for derivatives of linoleic and linolenic acids, aquilegia vulgaris oil for columbinic acid and sunflower oil, olive oil or safflower oil for derivatives of oleic acid, often together with linoleic acids. Other suitable oils are obtainable from hemp, which can be processed to derive stearadonic acid derivatives and maize corn oil. An especially convenient natural oil by virtue of its characteristics and availability comprises sunflower oil, ranging from those rich in oleic acid glycerides to those rich in linoleic acid glycerides, rich indicating that its content is higher than that of the other named acid.

Preferably the unsaturated oil is a natural oil which is still preferably selected from at least one of coriander seed oil, borage seed oil, evening primrose oil, maize corn oil, sunflower oil, safflower oil, algal oil or mixtures thereof. More preferred natural oils are sunflower oil, algal oil, preferably sunflower oil. The unsaturated oil is preferably included in an amount ranging from 0.05 wt.% to 10 wt.%, more preferably 1 wt.% to 5 wt.%% by weight of the composition.

The unsaturated oil is preferably present in an amount ranging from 0.05 wt.% to 10 wt.% in the antiperspirant composition. Preferably the antiperspirant composition comprises at least 0.08 wt.%, still preferably at least 0.1 wt.%, still preferably at least 0.3 wt.%, most preferably at least 0.5 wt.% of the metal based antiperspirant active, but typically not more than 8 wt.%, still preferably not more than 6 wt.%, still further preferably not more than 5 wt.%, still more preferably not more than 4 wt.% and most preferably not more than 3 wt.%, of an unsaturated oil based on the weight of the antiperspirant composition.

### Silicone non-ionic surfactant

According to the first aspect of the present invention disclosed antiperspirant composition includes a silicone non-ionic surfactant having an HLB value of less than 11. The antiperspirant composition includes from 1 wt.% to 20 wt.% silicone non-ionic surfactant.

As used herein, the term "HLB" refers to the "hydrophilic-lipophilic balance" of a molecule, such as a surfactant. The HLB number increases with increasing hydrophilicity. The HLB system is a semi-empirical method to predict what type of surfactant properties a molecular structure will provide. The HLB system is based on the concept that some molecules have hydrophilic groups, other molecules have lipophilic groups, and some have both. The HLB of a surfactant can be calculated according to Griffin WC: "Classification of Surface- Active Agents by 'HLB,'" Journal of the Society of Cosmetic Chemists 1 (1949): 311; and Griffin WC: "Calculation of HLB Values of Non-Ionic Surfactants," Journal of the Society of Cosmetic Chemists 5 (1954): 259. The HLB of a mixture of surfactants may be calculated by multiplying the proportion of each surfactant in the mixture by its HLB value and adding up the resulting values, as is known in the art.

The HLB value of the silicone nonionic surfactant is less than 11, still preferably the HLB value of the silicone non-ionic surfactant ranges from 2 to 10. HLB is the hydrophilic lipophilic balance which is calculated using the Griffin method wherein HLB = 20 x Mh / M wherein Mh is the molecular mass of the hydrophilic portion of the molecule and M is the molecular mass of the whole molecule, giving a result on an arbitrary scale of 0 to 20.

The nonionic silicone surfactant is preferably a dimethicone based silicone surfactant. Dimethicones are methyl substituted polyorganosiloxanes.

Preferred nonionic silicone surfactant includes silicone copolyols where polyethylene glycol and polypropylene glycol are copolymerized with dimethicone either individually or together. These combinations may be either graft, block, or end terminated. Graft polymers are those where the EO or PO chains are attached at various points along the main silicone backbone. Block or end terminated polymers have the EO or PO chains at the end of the silicone backbone. Preferably the chains may be either mixed or homopolymers. The preferred number of repeating units is below 20 and most preferred below 18.

Preferably the nonionic silicone surfactant is an alkyldimethicone copolyol corresponding to the formula (I) below:

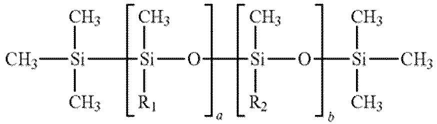

Wherein;
R₁ denotes a linear or branched, C₁-C₂₀, preferably C₁-C₁₆, alkyl group
R₂ denotes the group: CₙH2ₙ-(-OC₂H₄-)ₓ-(-OC₃H₆-)_{y}-O-R₃;
R₃ denotes a hydrogen atom or linear or branched alkyl radical containing from 1 to 12 carbon atoms;
a is an integer ranging from 1 to 500
b denotes an integer ranging from 1 to 500
n is integer ranging from 2 to 12, and preferably from 2 to 5
x denotes an integer ranging from 1 to 50
y denotes an integer ranging from 0 to 50
with the proviso that, when y is other than zero, the ratio x/y is greater than 1, and preferably ranges from 2 to 11.

Among the alkyl dimethicone copolyols of formula (I) the preferred includes cetyl PEG/PPG-10/1 dimethicone, and more particularly the cetyl PEG/PPG-10/1 dimethicone and dimethicone (INCI name) mixture, such as the product sold under the trade name Abil EM90 by the company Evonik Industries.

Preferred nonionic silicone surfactant may have the general formula

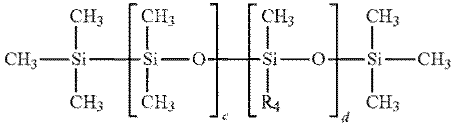

Wherein;
R₄ denotes the group CₘH₂ₘ-(-OC₂H₄-)ₛ-(-OC₃H₆-)ₜ-O-R₅;
R₅ denotes a hydrogen atom or linear or branched alkyl radical containing from 1 to 12 carbon atoms;
c is an integer ranging from 1 to 500
d denotes an integer ranging from 1 to 500
m is integer ranging from 2 to 12, and preferably from 2 to 5
s denotes an integer ranging from 1 to 50
t denotes an integer ranging from 0 to 50
with the proviso that the sum of s+t is greater than or equal to 1.

Among the dimethicone copolyols of formula (II) the preferred includes PEG-18/PPG-18 dimethicone, and more particularly the cyclopentasiloxane (and) PEG-18/PPG-18 dimethicone (INCI name) mixture, such as the product sold by the Dow Chemicals under the trade name Dowsil 5225 C.

The dimethicone based nonionic silicone surfactant may also include those under the general formula (III) 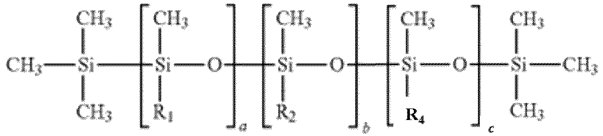
R₁ denotes a linear or branched, C₁-C₂₀, preferably C₁-C₁₆, alkyl group
R₂ denotes the group: CₙH2ₙ-(-OC₂H₄-)ₓ-(-OC₃H₆-)_{y}-O-R₃;
R₃ denotes a hydrogen atom or linear or branched alkyl radical containing from 1 to 12 carbon atoms;
R₄ denotes CₘH2ₘ-R₅
R₅ denotes hydrogen atom or Si-(O-R₆)₃
R₆ denotes a linear or branched, C₁-C₁₀, preferably C₁-C₅, alkyl group
a is an integer ranging from 1 to 500
b denotes an integer ranging from 1 to 500
c denotes an integer ranging from 0 to 500, b+c ¹ #4
n is integer ranging from 2 to 12, and preferably from 2 to 5
m is an integer ranging from 2 to 4
x denotes an integer ranging from 1 to 50
y denotes an integer ranging from 0 to 50
with the proviso that, when y is other than zero, the ratio x/y is greater than 1, and preferably ranges from 2 to 11.

More preferably the nonionic silicone surfactant is a selected from the non-limiting examples which includes Lauryl PEG-8-Dimethicone sold by Siltech Corporation under the tradename Silube J208-412 (HLB value 8 to 10), Lauryl-PEG-10-tris(trimethylsiloxy)silylethyldimethicone sold by Dow chemicals under the trade name Dowsil ES 5300 (HLB value 3), PEG/PPG-18/18 dimethicone (and) cyclopentasiloxane, bis-PEG/PPG-14/14Dimethicone (and) dimethicone, and more particularly the bis-PEG-14/PPG-14 dimethicone (and) cyclopentasiloxane (INCI name) mixture, such as the product sold by the Evonik under the trade name Abil EM 97, (HLB value 5), PEG/PPG-18/18 Dimethicone (and) cyclopentasiloxane, commercially available in a mixture with cyclopentasiloxane (tradename Dowsil 5225C), cetyl PEG/PPG-10/1 Dimethicone (tradename Abil^{®} EM 90, with HLB value 5), all commercially available from the respective companies as mentioned.

The nonionic silicone surfactant may preferably be a PEG-x- Dimethicone with x ranging from 1 to 18, still preferably from 1 to 10.

The composition according to the present invention may include other silicone-based compound for example cyclopentasiloxane (not a nonionic surfactant in accordance with the present invention), however when present the amount of the other silicone-based compound present in the antiperspirant composition is lower than the amount of the metal based antiperspirant active. More preferably the weight ratio of the antiperspirant active to other silicone-based compound present in the composition is 2:1 or higher.

### Antiperspirant composition

The antiperspirant composition according to the first aspect of the invention is an anhydrous antiperspirant composition. The antiperspirant composition is preferably delivered as a liquid, lotion, cream, foam, scrub, gel or stick form. Alternately it may be delivered through a roll-on device or using a propellant containing aerosol can. Most preferably the composition is delivered in stick form, through a roll-on device or by an aerosol can.

Preferably the composition of the invention comprises a topically acceptable carrier. The topically acceptable carrier is preferably anhydrous. By an anhydrous carrier is meant that water content in the composition is less than 10 wt%, preferably less than 5 wt%, more preferably less than 2 wt%, still more preferably less than 1 wt.% and optimally absent from the antiperspirant composition. To enable this, the anhydrous carrier preferably comprises a silicone compound other than the silicone nonionic surfactant, an alcohol or a wax. The alcohol, when used, could be a low boiling (C₂-C₄) alcohol or a polyhydric alcohol, preferably a polyhydric alcohol.

The pH of the antiperspirant composition is preferably higher than 3.5, more preferably in the range of 4 to 7. The pH of the composition of the invention is measured using the following procedure: Equal volumes of the composition and model ionic sweat (pH 6.1) are mixed, and the pH 5 value is measured using an accurate range pH test paper.

The antiperspirant composition includes less than 10 wt.% water preferably less than 5 wt%, more preferably less than 2 wt%, still more preferably less than 1 wt.% and optimally water is absent from the antiperspirant composition.

Preferably the weight ratio of the antiperspirant active to the silicone non-ionic surfactant in the composition ranges from 1.5:1 to 8:1, still preferably from 1.5: to 7.8:1, still further preferably from 1.5:1 to 3:1, more preferably from 1.75:1 to 2:1.

### Optional ingredients

The compositions according to the invention may optionally contain additives customary in cosmetics, for example, perfume, thickeners, deodorants, antimicrobial substances, regreasing agents, complexing and masking agents, pearlescent agents, plant extracts, vitamins, active ingredients, preservatives, bactericides, dyes, pigments that have a coloring effect, thickeners, moisturizing and/or humectant substances, fats, oils, waxes or other usual constituents of a cosmetic or dermatological formulation, such as alcohols, polyols, polymers, foam stabilizers, electrolytes, organic solvents or silicone derivatives, unless they are counter to the composition according to the invention and the use thereof.

### Moisturizers:

Moisturizers can also advantageously be used as anti-wrinkle substances for protection against aging of the skin, such as occur, e.g., in skin aging.

### Antioxidant compound:

The antiperspirant composition may include an antioxidant compound. Suitable and conventional antioxidants which are generally included in such compositions may be added in the present invention. Such antioxidants are selected from one or more of butyl hydroxy toluene or its derivatives, pentaerythrityl tetra-di-t-butyl hydroxyhydrocinnamate (sold as Tinogard TT), or dilauryl thiodipropionate (sold as Tinogard DA), Tetramethylhydroxypiperidinol) Citrate (sold as Tinogard Q), tocopherol acetate or ascorbic acid and its derivatives. The antiperspirant composition preferably comprises 0.001 wt.% to 10 wt.%, more preferably 0.01 wt. to 5 wt.%, and most preferably 0.01 wt. to 2 wt.% antioxidant compound by weight of the composition.

The antiperspirant composition in accordance with the present invention preferably include one or more optional ingredients selected from polyhydric alcohol.

Preferably the antiperspirant composition according to the present invention comprises a polyhydric alcohol. Polyhydric alcohol is also referred to in short as polyol. A polyhydric alcohol as per the present invention is a compound having two or more hydroxyl groups. Suitable class of polyhydric alcohols that may be included in the composition of the invention are monomeric polyols, polyalkylene glycols or sugars. Preferred monomeric polyols are glycol; alkylene glycol e.g. propylene glycol; glycerol; or xylitol, more preferably propylene glycol.

Suitable polyalkylene glycols are polyethylene glycol or polypropylene glycol. Sugars for inclusion in the invention could be monomeric, dimeric, trimeric or of the polymeric form. Preferred sugars include glucose, fructose, mannose, sucrose, threitol, erythritol, sorbitol, mannitol, galactitol, adonitol, dextran, or cyclodextrin. Of these the more preferred sugars are glucose, fructose, sucrose, sorbitol, mannitol, adonitol, dextran, or cyclodextrin.

Other components commonly included in conventional antiperspirant compositions may also be incorporated in the composition of the present invention. Such components include skin care agents such as emollients, humectants and skin barrier promoters; skin appearance modifiers such as skin lightening agents and skin smoothing agents; antimicrobial agents, in particular organic anti-microbial agents, and preservatives.

### Method of application

The antiperspirant composition can be applied cosmetically and topically to the skin, broadly speaking, by one of two methods. Some consumers prefer one method and some others, the other method. In one method, sometimes called a contact method, a composition is wiped across the surface of the skin, depositing a fraction of the composition as it passes. In the second method, sometimes called the non-contact method, the composition is sprayed from a dispenser held proximate to the skin, often in The antiperspirant composition can be applied cosmetically and topically to the skin, broadly speaking, by one of two methods. Some consumers prefer one method and 30 some others, the other method. In one method, sometimes called a contact method, a composition is wiped across the surface of the skin, depositing a fraction of the composition as it passes. In the second method, sometimes called the non-contact method, the composition is sprayed from a dispenser held proximate to the skin, often in an area of about 10 to 20 cm². The spray can be developed by mechanical means of generating pressure on the contents of the dispenser, such as a pump or a squeezable sidewall or by internally generated pressure arising from a fraction of a liquefied propellant volatilising, the dispenser commonly being called an aerosol.

There are broadly speaking two classes of contact compositions, one of which is liquid and usually applied using a roll-on dispenser or possibly absorbed into or onto a wipe, and in the second of which the antiperspirant active is distributed within a carrier liquid that forms a continuous phase that has been gelled. In one variation, the carrier fluid comprises a solvent for the antiperspirant and in a second variation, the antiperspirant remains a particulate solid that is suspended in an oil, usually a blend of oils.

The composition of the invention is preferably delivered as a composition in aerosol form or in stick form.

### Stick or soft solid compositions

Many different materials have been proposed as gellant for a continuous oil phase, including waxes, small molecule gelling agents and polymers. They each have their advantages and of them, one of the most popular class of gellant has comprised waxes, partly at least due to their ready availability and ease of processing, including in particular linear fatty alcohol wax gellants. A gelled antiperspirant composition is applied topically to skin by wiping it across and in contact with the skin, thereby depositing on the skin a thin film. The nature of the film depends to a significant extent on the gellant that is employed. Although wax fatty alcohols have been employed as gellant for many years, and are effective for the purpose of gelling, the resultant product is rather ineffective at improving the visual appearance of skin, and in particular underarm skin, to which the composition has been applied. This problem has been solved by including ameliorating materials for example, di or polyhydric humectants and/or a triglyceride oil.

### Roll-on compositions

Liquid compositions that are applicable from a roll-on broadly speaking can be divided into two classes, namely those in which an antiperspirant active is suspended in a hydrophobic carrier, such as a volatile silicone and those in which the antiperspirant active is dissolved in a carrier liquid. The latter has proven to be more popular. There are mainly two sorts of dissolving carrier liquid, namely carriers that are predominantly alcoholic, which is to say the greater part of the dissolving carrier fluid comprises ethanol and the second class in which the carrier liquid is mainly water. The former was very popular because ethanol is a mild bactericide in its own right.

### Aerosol compositions

The antiperspirant composition may be delivered through an aerosol composition which comprises a propellant in addition to the other ingredients described hereinabove. Commonly, the propellant is employed in a weight ratio to the base formulation of from 95:5 to 5:95. Depending on the propellant, in such aerosol compositions the ratio of propellant to base formulation is normally at least 20:80, generally at least 30:70, particularly at least 40:60, and in many formulations, the weight ratio is from 90:10 to 50:50. A ratio range of from 70:30 to 90:10 is sometimes preferred.

Propellants herein generally are one of three classes; i) low boiling point gasses liquifided by compression, ii) volatile ethers and iii) compressed non-oxidising gases.

Class i) is conveniently a low boiling point material, typically boiling below -5°C, and often below -15°C, and in particular, alkanes and/or halogenated hydrocarbons. This class of propellant is usually liquefied at the pressure in the aerosol canister and evaporates to generate the pressure to expel the composition out of the canister. Examples of suitable alkanes include particularly propane, butane or isobutane. The second class of propellant comprises a very volatile ether of which the most widely employed ether hitherto is dimethyl ether. This propellant can advantageously be employed at relatively low weight ratio of propellant to base formulation, for example to as low as 5:95. It can also be employed in admixture with, for example, compressible/liquefiable alkane gasses. The third class of propellant comprises compressed non-oxidizing gasses, and in particular carbon dioxide or nitrogen. Inert gases like neon are a theoretical alternative.

When the composition of the invention is delivered in a roll-on, a firm solid or a stick format, the topically acceptable carrier comprises a hydrophobic carrier or an aqueous carrier. The hydrophobic carrier in such cases may comprise a silicone compound, low boiling alcohol or a wax. When the composition comprises a propellant it is delivered as an aerosol.

The composition of the present invention can comprise a wide range of other optional components. The CTFA Personal care Ingredient Handbook, Second Edition, 1992, which is incorporated by reference herein in its entirety, describes a wide variety of non-limiting personal care and pharmaceutical ingredients commonly used in the skin care industry, which are suitable for use in the compositions of the present invention. Examples includes: binders, biological additives, buffering agents, colorants, thickeners, polymers, astringents, fragrance, conditioners, exfoliating agents, pH adjusters, preservatives, natural extracts, essential oils, skin sensates, skin soothing agents, and skin healing agents.

According to another aspect of the present invention there is provided a method of minimizing staining or yellowish colouration of fabric comprising the steps of (a) applying a composition of the invention onto a body part, wherein the composition applied on to a body part comes in contact with the fabric when worn by an individual, preferably the axilla of an individual followed by (b) washing, (c) rinsing and (d) drying the fabric.

The composition is preferably applied on the axilla. The method is non-therapeutic or for cosmetic application.

Yet another aspect of the present invention relates to use of nonionic silicone surfactant having an HLB value of less than 11 for manufacture of a composition of the invention, for minimizing staining of fabrics which comes in contact with the composition. The fabric may be part of a clothing which are worn by individuals who use the composition on their body part like the axila which comes in contact with the fabric.

According to another aspect, the present invention relates to use of 1 wt.% to 20 wt.% nonionic silicone surfactant having a HLB value of less than 11 in an antiperspirant composition comprising (i) a metal based antiperspirant active (ii) an unsaturated oil having an iodine value of at least 100 and (iii) less than 10 wt.% water for minimizing staining of fabrics which come in contact with the composition.

According to another aspect, the present invention relates to use of 1 wt.% to 20 wt.% nonionic silicone surfactant having a HLB value of less than 11 in an antiperspirant composition comprising (i) a metal based antiperspirant active (ii) an unsaturated oil having an iodine value of at least 100 and (iii) less than 10 wt.% water for reducing the consumer perception of white marks on fabric which come in contact with the composition, particularly dark coloured fabric, more particularly black fabric.

### Examples

The invention will now be illustrated with the help of the following non-limiting examples.

Anhydrous antiperspirant aerosol composition as shown in Table 1 below were prepared:

**Table 1**

| Ingredient | wt.% |
|---|---|
| Cyclopentasiloxane | 2.5 |
| PPG 14 Butyl ether | 3 |
| Sunflower seed oil (unsaturated oil) | 0.5 |
| BHT | 0.1 |
| Aluminium chlorohydrate (metal based antiperspirant active) | 5.5 |
| Disteardimonium hectorite | 0.6 |
| Propylene carbonate | 0.015 |
| C₁₂₋₁₅ alkyl benzoate | 0.5 |
| Octyldodecanol | 0.12 |
| Non-ionic silicone surfactant | See Table 2 and 5 below for type and concentration; |
| Butane, isobutane, propane | to 100 |

### Example 1: Effect of presence of a non-ionic silicone surfactant in accordance to the present invention on stain removal

The effect of inclusion of different types of non-ionic silicone surfactants as in Table 2 and Table 3 in the composition as given in Table 1 was studied, and the cleaning efficacy was measured as given in the protocol below.

### Protocol used for the evaluation:

In this study, a 10 X 10 cm² fabric swatch was taken. The central circular area corresponding to 33 cm² was applied with 0.3 g of APA base formulation containing 0.05 g model sebum mix and 0.5 ml of artificial sweat. Study was done with five replicates. The treated swatches were incubated in a hot air oven at 45 °C for 12 hours.

Treated fabrics were washed in an IFB front loader washing machine, programmed for wash and two rinses. Treated fabrics were stapled to clean, air dry polycotton ballast to obtain an approximate load of 2 ±0.1 kg and placed into the washing machine. The hardness of water was maintained at 24 fH water 2:1, Ca: Mg. 36 g of Persil Non-Bio Liquid detergent was added in the dosing chamber. The swatches were dried using line drying at room temperature (25 °C) for 10 days.

The above process of treating the swatches followed by washing the swatches was repeated 4 times and after the final cycle the chromophoric evaluation of the swatches was conducted using a Konika Minolta spectrophotometer Model-2600d. A Konika Minolta spectrophotometer Model-2600d was used to measure the stain intensity on the swatches. L*, a*, b* were measured at three different areas of the stained and untreated fabrics. Δ b* and Δ E* were calculated with respect to artificial sebum and artificial sweat treated fabric, undergoing the same treatment conditions as the baseline.

The lower the Δ E* the better is the overall whiteness and therefore lower Δ E* values indicate better stain removal performance. Similarly, higher is the "Change in ΔE* from Control [ΔE*_{control} - ΔE*_{sample]"}, better is the overall whiteness and better stain removal performance, where the sample refers to either Ex 1 or Ex 2.

**Table 2**

| Example | Non-ionic surfactant | (wt%) in composition of table 1 | HLB | ΔE* after 4 wash cycle | Change in ΔE* from Control [ΔE*_{control} - ΔE*_{sample]} |
|---|---|---|---|---|---|
| Control | -Nil- | 0 | - | 7.5 | - |
| Ex 1 | Bis-PEG/PPG-14/14 Dimethicone¹ | 2 | 5 | 3.8 | 3.7 |
| Ex 2 | Cetyl PEG/PPG-10/1 Dimethicone² | 2 | 5 | 5.3 | 2.2 |

| | | | | | |
|---|---|---|---|---|---|
| ¹ sold by Evonik Industries under the tradename Abil EM 97/Abil EM97 S; ² sold by Evonik Industries under the tradename Abil EM 90 | | | | | |

The data in the table 2 shows that the two compositions according to the present invention (Ex 1 and Ex 2) comprising a non-ionic silicone surfactant having HLB less than 11 delivers much better whitening as evidenced by a lowered ΔE* value as compared to a control composition which does not include a non-ionic surfactant.

### Example 2: Effect of the HLB of the surfactant on the whiteness performance

Different anhydrous antiperspirant composition as provided in table 3 were evaluated using the protocol described in Example 1 and after the 4 wash cycle the ΔE* values were recorded and is provided in table 3 below.
- Control - is the commercially available Dove original antiperspirant composition (antiperspirant active level 5 wt.% and sunflower seed oil 0.5 wt.%.
- Ex A - is the commercially available Dove original antiperspirant composition where 2 wt.% of XIAMETER^{™} OFX-193 (PEG-12 dimethicone with HLB value 12 sold by Dow Chemicals) is added and accordingly adjusted with butane/isobutane/propane.
- Ex 3 - is the commercially available Dove original where 2 wt.% of Abil^{®} EM 97 S ( Bis-PEG/PPG-14/14 Dimethicone and dimethicone with HLB value 5 sold by Evonik Industries) is added and accordingly adjusted with butane/isobutane/propane.
In the below, the term sample refers to either Ex A or Ex 3.

**Table 3**

| Formulation | Nonionic silicone surfactant (HLB value) | Nonionic silicone surfactant (wt. %) | ΔE* (after 4 wash cycles) | Change in ΔE* from Control [ΔE*_{control} - ΔE*_{sample]} |
|---|---|---|---|---|
| Control | - | 0 | 4.40 | 0 |
| Ex A | PEG-12 dimethicone (12) | 2 | 2.49 | 1.9 |
| Ex 3 | Bis-PEG/PPG-14/14 Dimethicone (5) | 2 | 1.02 | 3.4 |

The data in the table 3 shows that when a nonionic silicone surfactant with an HLB value outside the range is added to an anhydrous antiperspirant composition (Ex A) the whiteness performance of the composition is much lower as compared to a composition which includes a nonionic silicone surfactant with an HLB value within the claimed range (i.e; less than 11, in Ex 3 the HLB value is 5). This is reflected in the higher values of the change in ΔE* from control for the example according to the present invention (3.4) as compared to the comparative example (Ex A, 1.9).

### Example 3: Evaluation of the nonionic silicone surfactant and other nonionic surfactant with HLB less than 11 on the whiteness performance

Different anhydrous antiperspirant compositions as provided in table 4 were evaluated using the protocol described in Example 1 and after the 4 wash cycle the ΔE* values were recorded and is provided in table 3 below.
- Control - is the commercially available Dove original antiperspirant composition (antiperspirant active level 5 wt.% and sunflower seed oil 0.5 wt.%).
- Ex 1 - is the commercially available Dove original where 2 wt.% of Abil^{®} EM 97 S (Bis-PEG/PPG-14/14 Dimethicone and dimethicone with HLB value 5 sold by Evonik Industries) is added and accordingly adjusted with butane/isobutane/propane.
- Ex 2 - is the commercially available Dove original antiperspirant composition where 2 wt.% of Abil^{®} EM 90 (with HLB 5, sold by Evonik) is added and accordingly adjusted with butane/isobutane/propane.
- Ex B - is the commercially available Dove original antiperspirant composition where 2 wt.% of Isolan 17MB (with HLB 5, sold by Evonik) is added and accordingly adjusted with butane/isobutane/propane.
- Ex C - is the commercially available Dove original antiperspirant composition where 2 wt.% of Isoamyl laurate (with HLB 5, sold by Evonik under the tradename Dermafeel Sensolve) is added and accordingly adjusted with butane/isobutane/propane.

**Table 4**

| Example | Non-ionic surfactant | (wt%) in composition of table 1 | HLB | Change in ΔE* from Control [ΔE*_{control} - ΔE*_{sample]} |
|---|---|---|---|---|
| Control | -Nil- | 0 | - | - |
| Ex 1 | Bis-PEG/PPG-14/14 Dimethicone¹ | 2 | 5 | 3.7 |
| Ex 2 | Cetyl PEG/PPG-10/1 Dimethicone² | 2 | 5 | 2.2 |
| Ex B | Polyglyceryl-4 Diisostearate/Polyhydroxystearate/ Sebacate (and) Caprylic/Capric Triglyceride (and) Polyglyceryl-3 Oleate (and) Diisostearoyl Polyglyceryl-3 Dimer Dilinoleate³ | 2 | 5 | 0.9 |
| Ex C | Isoamyl laurate⁴ | 2 | 5 | -0.1 |

| | | | | |
|---|---|---|---|---|
| ¹ sold by Evonik Industries under the tradename Abil EM 97/Abil EM97 S; ² sold by Evonik Industries under the tradename Abil EM 90; ³ sold by Evonik Industries under the tradename Isolan 17MB ⁴ sold by Evonik Industries under the tradename Dermafeel Sensolve. | | | | |

The data in table 4 shows that the compositions according to the present invention (Ex 1 and Ex 2) comprising a non-ionic silicone surfactant having HLB less than 11 delivers much better overall whiteness and better stain removal performance (as evidenced by a higher value for the change in ΔE* from control) as compared to comparative compositions which has non-ionic surfactant (other than the silicone surfactant) with HLB less than 11 (as evidenced by a comparatively lower value for the change in ΔE* from control in Ex B and Ex C).

### Example 3: Evaluation of the presence of the nonionic silicone surfactant in accordance to the present invention on the appearance of white stains on black fabrics

The effect of inclusion of different types of non-ionic silicone surfactants as in Table 5 in the composition as given in Table 1 was studied, and the cleaning efficacy was measured as given in the protocol below. Control_{A} was used for evaluating the first set of Ex 1 and Ex 4 and the Controls was used for evaluating the first set of Ex 2 and Ex 5.

### Protocol used for the evaluation:

A. Immediate Whiteness on black fabric: In this study, 0.5 grams of the base composition (of the aerosol composition provided in table 1 minus the propellant) was uniformly smeared over a black fabric swatch in a circular area of 66 cm² and the composition was allowed to stand at room temperature for 15 minutes. At the end of the 15 minutes the chromophoric evaluation of the swatches was conducted using a Konika Minolta spectrophotometer Model-2600d. A Konika Minolta spectrophotometer Model-2600d was used to measure the stain intensity on the swatches. L* values were measured at three different areas of the stained and untreated fabrics and was recorded and provided in table 5.
B. Whiteness of black fabric after 4 wash cycles: In this study, 0.5 grams of the base composition (of the aerosol composition provided in table 1 minus the propellant) was uniformly smeared over a black fabric swatch in a circular area of 66 cm² and the composition was allowed to stand at room temperature and aged in a hot air oven at 45 °C for 12 hours. After this, the treated black swatches were washed using the protocol followed in Example 1. At the final wash cycle (4 wash cycles) the chromophoric evaluation of the swatches was conducted using a Konika Minolta spectrophotometer Model-2600d. A Konika Minolta spectrophotometer Model-2600d was used to measure the stain intensity on the swatches. L* values were measured at three different areas of the stained and untreated fabrics and was recorded and provided in table 5.
Lower L* value indicates lower white marks deposited on the fabrics.

**Table 5**

| Example | Non-ionic surfactant | (wt%) in composition of table 1 | L* (whiteness) | Std. | L* (whiteness) | Std. |
|---|---|---|---|---|---|---|
| | | | Immediate | Dev. | After washing | Dev. |
| Control_{A} | -Nil- | 0 | 49 | 0.6 | 42 | 0.7 |
| Ex 1 | Bis-PEG/PPG-14/14 Dimethicone¹ | 2 | 42 | 0.4 | 29 | 0.9 |
| Ex 4 | Bis-PEG/PPG-14/14 Dimethicone¹ | 6 | 26 | 0.6 | 18 | 0.7 |
| | | | | | | |
| Controls | -Nil- | 0 | 39 | 0.5 | 38 | 0.5 |
| Ex 2 | Cetyl PEG/PPG-10/1 Dimethicone² | 2 | 34 | 0.4 | 26 | 0.9 |
| Ex 5 | Cetyl PEG/PPG-10/1 Dimethicone² | 6 | 22 | 0.4 | 17 | 0.8 |

| | | | | | | |
|---|---|---|---|---|---|---|
| ¹ sold by Evonik Industries under the tradename Abil EM 97/Abil EM97 S; ² sold by Evonik Industries under the tradename Abil EM 90. | | | | | | |

The data provided in table 5 indicates that anhydrous antiperspirant composition comprising the nonionic silicone surfactant according to the present invention effectively removes the patchy white marks formed by the antiperspirant composition on the black fabrics. This benefit is seen both when the black fabric is in contact with the composition for short duration as well as when the fabric is washed.

These white marks which are consumer perceivable were found to be significantly reduced when nonionic silicone surfactant according to the present invention are present within the claimed ranges (both 2 wt.% and 6 wt.%).

It was also observed that the non-ionic surfactants can effectively remove the white marks (which exhibits a patchy appearance on the fabric) after the wash cycle, and when the surfactants are present at levels higher than 5 wt.% reduction in the perception of whiteness from fabrics is instantaneous (in the first wash).

## Claims

1. An anhydrous antiperspirant composition comprising:
(i) a metal based antiperspirant active;
(ii) 1 wt.% to 20 wt.% silicone non-ionic surfactant having an HLB value of less than 11;
(iii) an unsaturated oil having an iodine value of at least 100;
(iv) less than 10 wt.% water.

2. A composition according to claim 1 wherein the weight ratio of the antiperspirant active to the silicone non-ionic surfactant in the composition ranges from 1.5:1 to 8:1, preferably from 1.5:1 to 3:1, more preferably from 1.75:1 to 2:1.

3. A composition according to claim 1 or 2 wherein the metal based antiperspirant active is present in an amount ranging from 2 wt.% to 30 wt.% in the composition.

4. A composition according to claim 1 wherein the silicone non-ionic surfactant is a dimethicone based nonionic silicone surfactant may also include those under the general formula (III) 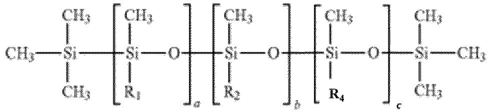
R₁ denotes a linear or branched, C₁-C₂₀, preferably C₁-C₁₆, alkyl group
R₂ denotes the group: CₙH2ₙ-(-OC₂H₄-)ₓ-(-OC₃H₆-)_{y}-O-R₃;
R₃ denotes a hydrogen atom or linear or branched alkyl radical containing from 1 to 12 carbon atoms;
R₄ denotes CₘH2ₘ-R₅
R₅ denotes hydrogen atom or Si-(O-R₆)₃
R₆ denotes a linear or branched, C₁-C₁₀, preferably C₁-C₅, alkyl group
a is an integer ranging from 1 to 500
b denotes an integer ranging from 1 to 500
c denotes an integer ranging from 0 to 500, b+c ≥ 1
n is integer ranging from 2 to 12, and preferably from 2 to 5
m is an integer ranging from 2 to 4
x denotes an integer ranging from 1 to 50
y denotes an integer ranging from 0 to 50
with the proviso that, when y is other than zero, the ratio x/y is greater than 1, and preferably ranges from 2 to 11.

5. A composition according to any one of the preceding claims wherein the dimethicone based nonionic silicone surfactant is selected from the group consisting of Lauryl-PEG-8-dimethicone, Lauryl-PEG-10-tris(trimethylsiloxy)silylethyldimethicone, PEG/PPG-18/18 dimethicone, PEG/PPG-18/18 dimethicone (and) cyclopentasiloxane, bis-PEG-14/PPG-14 Dimethicone (and) cyclopentasiloxane, Cetyl PEG/PPG-10/1 Dimethicone or mixtures thereof.

6. A composition according to any one of the preceding claims wherein the HLB value of the silicone non-ionic surfactant ranges from 2 to 10.

7. A composition as claimed in any one of the preceding claims wherein the metal based antiperspirant active is selected from an aluminium, zirconium or mixed aluminium/zirconium salt, preferably, aluminium chlorohydrate, aluminium-zirconium tetrachlorohydrex glycine complex, aluminium-zirconium octachlorohydrex glycine complex, aluminium-zirconium pentachlorohydrate, aluminium sesquichlorohydrate or mixtures thereof.

8. A composition as claimed in any one of the preceding claims wherein the unsaturated oil is selected from at least one of coriander seed oil, borage seed oil, evening primrose oil, maize corn oil, sunflower oil, safflower oil, and algal oil, preferably sunflower seed oil.

9. A composition as claimed in any one of the preceding claims comprising an antioxidant selected from butyl hydroxy toluene, pentaerythrityl tetra-di-t-butyl hydroxyhydrocinnamate (sold as Tinogard TT), or dilauryl thiodipropionate (sold as Tinogard DA).

10. A composition according to any one of the preceding claims wherein the unsaturated oil is present in an amount ranging from 0.05 wt.% to 10 wt.% in the composition.

11. A composition as claimed in any one of the preceding claims comprising 0.01 wt.% to 2 wt.% antioxidant compound by weight of the composition.

12. An antiperspirant composition as claimed in any one of the preceding claims which is delivered in a stick form, gel, roll-on or in an aerosol can.

13. A method of minimizing staining or yellowish coloration of fabric worn by an individual comprising the steps of (a) applying a composition as claimed in any one of the preceding claims on to a body part, wherein the composition applied on to a body part comes in contact with the fabric when worn by an individual, preferably the axilla of the individual followed by (b) washing, (c) rinsing and (d) optionally drying the fabric.

14. Use of 1 wt.% to 20 wt.% nonionic silicone surfactant having a HLB value of less than 11 in an antiperspirant composition comprising (i) a metal based antiperspirant active (ii) an unsaturated oil having an iodine value of at least 100 and (iii) less than 10 wt.% water for minimizing staining of fabrics which come in contact with the composition.

15. Use of 1 wt.% to 20 wt.% nonionic silicone surfactant having a HLB value of less than 11 in an antiperspirant composition comprising (i) a metal based antiperspirant active (ii) an unsaturated oil having an iodine value of at least 100 and (iii) less than 10 wt.% water for reducing the consumer perception of white marks on fabric which come in contact with the composition.

## Patentansprüche

1. Wasserfreie schweißhemmende Zusammensetzung, umfassend;
(i) einen schweißhemmenden Wirkstoff auf Metallbasis;
(ii) 1 bis 20 Gew.-% nichtionisches Silicontensid mit einem HLB-Wert von weniger als 11;
(iii) ein ungesättigtes Öl mit einer Jodzahl von mindestens 100;
(iv) weniger als 10 Gew.-% Wasser.

2. Zusammensetzung nach Anspruch 1, wobei das Gewichtsverhältnis des schweißhemmenden Wirkstoffs zu dem nichtionischen Silicontensid in der Zusammensetzung im Bereich von 1,5:1 bis 8:1, vorzugsweise von 1,5:1 bis 3:1, bevorzugter von 1,75:1 bis 2:1 liegt.

3. Zusammensetzung nach Anspruch 1 oder 2, wobei der schweißhemmende Wirkstoff auf Metallbasis in einer Menge von 2 bis 30 Gew.-% in der Zusammensetzung vorliegt.

4. Zusammensetzung nach Anspruch 1, wobei das nichtionische Silicontensid ein nichtionisches Silicontensid auf Dimethiconbasis ist, aber auch solche der allgemeinen Formel (III) 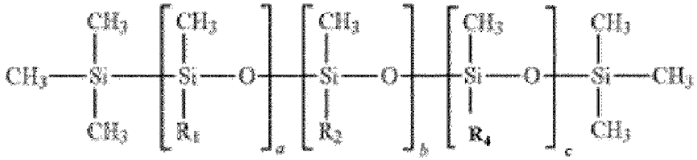 umfassen kann,
worin bedeuten:
R₁ eine lineare oder verzweigte C₁-C₂₀-, vorzugsweise C₁-C₁₆-Alkylgruppe;
R₂ die Gruppe: CₙH₂ₙ-(-OC₂H₄-)ₓ-(-OC₃H₆-)_{y}-O-R₃;
R₃ ein Wasserstoffatom oder einen linearen oder verzweigten Alkylrest mit 1 bis 12 Kohlenstoffatomen;
R₄ CₘH₂ₘ-R5;
R₅ ein Wasserstoffatom oder Si-(O-R₆)₃;
R₆ eine lineare oder verzweigte C₁-C₁₀-, vorzugsweise C₁-C₅-Alkylgruppe;
a eine ganze Zahl in dem Bereich von 1 bis 500;
b eine ganze Zahl in dem Bereich von 1 bis 500;
c eine ganze Zahl in dem Bereich von 0 bis 500,
wobei b+c ≥ 1 sind;
n eine ganze Zahl in dem Bereich von 2 bis 12 und vorzugsweise von 2 bis 5;
m eine ganze Zahl in dem Bereich von 2 bis 4;
x eine ganze Zahl in dem Bereich von 1 bis 50;
y ist eine ganze Zahl in dem Bereich von 0 bis 50,
dies mit der Maßgabe, dass dann, wenn y von Null verschieden ist, das Verhältnis x/y größer als 1 ist und vorzugsweise in dem Bereich von 2 bis 11 liegt.

5. Zusammensetzung nach irgendeinem der vorhergehenden Ansprüche, wobei das nichtionische Silicontensid auf Dimethiconbasis aus der Gruppe ausgewählt ist, bestehend aus Lauryl-PEG-8-Dimethicon, Lauryl-PEG-10-Tris(trimethylsiloxy)silylethyldimethicon, PEG/PPG-18/18-Dimethicon, PEG/PPG-18/18-Dimethicon (und) Cyclopentasiloxan, Bis-PEG-14/PPG-14-Dimethicon (und) Cyclopentasiloxan, Cetyl-PEG/PPG-10/1-Dimethicon oder Mischungen davon.

6. Zusammensetzung nach irgendeinem der vorhergehenden Ansprüche, wobei der HLB-Wert des nichtionischen Silicontensids in dem Bereich von 2 bis 10 liegt.

7. Zusammensetzung nach irgendeinem der vorhergehenden Ansprüche, wobei der schweißhemmende Wirkstoff auf Metallbasis ausgewählt ist unter einem Aluminium-, Zirkonium- oder gemischten Aluminium/Zirkonium-Salz, vorzugsweise Aluminiumchlorhydrat, Aluminium-Zirkonium-Tetrachlorhydrex-Glycin-Komplex, Aluminium-Zirkonium-Octachlorhydrex-Glycin-Komplex, Aluminium-Zirkonium-Pentachlorhydrat, Aluminium-Sesquichlorhydrat oder Gemischen davon.

8. Zusammensetzung nach irgendeinem der vorhergehenden Ansprüche, wobei das ungesättigte Öl aus mindestens einem der folgenden Öle ausgewählt ist: Koriandersamenöl, Borretschsamenöl, Nachtkerzenöl, Maiskeimöl, Sonnenblumenöl, Safloröl und Algenöl, vorzugsweise Sonnenblumenkernöl.

9. Zusammensetzung nach irgendeinem der vorhergehenden Ansprüche, umfassend ein Antioxidationsmittel, ausgewählt unter Butylhydroxytoluol, Pentaerythrityl-tetra-di-t-butylhydroxyhydrocinnamat (verkauft als Tinogard TT) oder Dilaurlythiodipropionat (verkauft als Tinogard DA).

10. Zusammensetzung nach irgendeinem der vorhergehenden Ansprüche, wobei das ungesättigte Öl in einer Menge in dem Bereich von 0,05 Gew.-% bis 10 Gew.-% in der Zusammensetzung vorliegt.

11. Zusammensetzung nach irgendeinem der vorhergehenden Ansprüche, umfassend 0,01 Gew.-% bis 2 Gew.-% Antioxidansverbindung, bezogen auf das Gewicht der Zusammensetzung.

12. Schweißhemmende Zusammensetzung nach irgendeinem der vorhergehenden Ansprüche, die in Form eines Stifts, Gels, Roll-ons oder einer Aerosoldose abgegeben wird.

13. Verfahren zur Minimierung von Fleckenbildung oder gelblicher Färbung von Textilien, die von einer Person getragen werden, umfassend die Schritte:
(a) Auftragen einer Zusammensetzung, wie in irgendeinem der vorhergehenden Ansprüche beansprucht, auf einen Körperteil, vorzugsweise auf die Achselhöhle der Person, wobei die auf einen Körperteil aufgetragene Zusammensetzung mit dem Textil in Kontakt kommt, wenn es von einer Person getragen wird, gefolgt von
(b) Waschen,
(c) Spülen und
(d) gegebenenfalls Trocknen des Textils.

14. Verwendung von 1 bis 20 Gew.-% nichtionischem Silicontensid mit einem HLB-Wert von weniger als 11 in einer schweißhemmenden Zusammensetzung, umfassend (i) einen schweißhemmenden Wirkstoff auf Metallbasis, (ii) ein ungesättigtes Öl mit einer Iodzahl von mindestens 100 und (iii) weniger als 10 Gew.-% Wasser, zur Minimierung der Fleckenbildung von Textilien, die mit der Zusammensetzung in Kontakt kommen.

15. Verwendung von 1 bis 20 Gew.-% nichtionischem Silicontensid mit einem HLB-Wert von weniger als 11 in einer schweißhemmenden Zusammensetzung, umfassend (i) einen schweißhemmenden Wirkstoff auf Metallbasis, (ii) ein ungesättigtes Öl mit einer Iodzahl von mindestens 100 und (iii) weniger als 10 Gew.-% Wasser, zur Verringerung der Wahrnehmung weißer Flecken auf Textilien, die mit der Zusammensetzung in Kontakt kommen, durch den Verbraucher.

## Revendications

1. Composition antitranspirante anhydre comprenant :
(i) un principe actif antitranspirant à base de métal ;
(ii) 1 % en poids à 20 % en poids de tensioactif non-ionique siliconé ayant un indice HLB inférieur à 11 ;
(iii) une huile insaturée ayant un indice d'iode d'au moins 100 ;
(iv) moins de 10 % en poids d'eau.

2. Composition selon la revendication 1, dans laquelle le rapport en poids du principe actif antitranspirant au tensioactif non-ionique siliconé dans la composition est situé dans la plage allant de 1,5/1 à 8/1, de préférence de 1,5/1 à 3/1, mieux encore de 1,75/1 à 2/1.

3. Composition selon la revendication 1 ou 2, dans laquelle le principe actif antitranspirant à base de métal est présent dans la composition en une quantité située dans la plage allant de 2 % en poids à 30 % en poids.

4. Composition selon la revendication 1, dans laquelle le tensioactif non-ionique siliconé est un tensioactif siliconé non-ionique à base de diméthicone qui peut aussi englober ceux de formule générale (III) 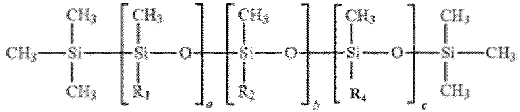
R₁ représente un groupe alkyle linéaire ou ramifié en C₁ à C₂₀, de préférence en C₁ à C₁₆ ;
R₂ représente le groupe CₙH₂ₙ-(-OC₂H₄-)ₓ-(-OC₃H₆-)_{y}-O-R₃ ;
R₃ représente un atome d'hydrogène ou un radical alkyle linéaire ou ramifié contenant 1 à 12 atomes de carbone ;
R₄ représente CₘH₂ₘ-R₅ ;
R₅ représente un atome d'hydrogène ou Si-(O-R₆)₃ ;
R₆ représente un groupe alkyle linéaire ou ramifié en C₁ à C₁₀, de préférence en C₁ à C₅ ;
a est un entier situé dans la plage allant de 1 à 500 ;
b est un entier situé dans la plage allant de 1 à 500 ;
c est un entier situé dans la plage allant de 0 à 500, et b + c ≥ 1 ;
n est un entier situé dans la plage allant de 2 à 12 et de préférence de 2 à 5 ;
m est un entier situé dans la plage allant de 2 à 4 ;
x est un entier situé dans la plage allant de 1 à 50 ;
y est un entier situé dans la plage allant de 0 à 50 ;
sous réserve que, lorsque y est différent de zéro, le rapport x/y soit supérieur à 1 et de préférence soit situé dans la plage allant de 2 à 11.

5. Composition selon l'une quelconque des revendications précédentes, dans laquelle le tensioactif siliconé non-ionique à base de diméthicone est choisi dans le groupe constitué par la lauryl-PEG-8-diméthicone, la lauryl-PEG-10-tris(triméthylsiloxy)silyléthyldiméthicone, la PEG/PPG-18/18 diméthicone, le PEG/PPG-18/18 diméthicone (et) cyclopentasiloxane, le bis-PEG-14/PPG-14 diméthicone (et) cyclopentasiloxane, la cétyl-PEG/PPG-10/1 diméthicone, et leurs mélanges.

6. Composition selon l'une quelconque des revendications précédentes, dans laquelle l'indice HLB du tensioactif non-ionique siliconé est situé dans la plage allant de 2 à 10.

7. Composition selon l'une quelconque des revendications précédentes, dans laquelle le principe actif antitranspirant à base de métal est choisi parmi les sels d'aluminium, de zirconium et mixtes d'aluminium/ zirconium, de préférence le chlorhydrate d'aluminium, le complexe d'aluminium-zirconium tétrachlorohydrex glycine, le complexe d'aluminium-zirconium octachlorhydrex glycine, le pentachlorhydrate d'aluminium-zirconium, le sesquichlorhydrate d'aluminium, et leurs mélanges.

8. Composition selon l'une quelconque des revendications précédentes, dans laquelle l'huile insaturée est au moins l'une choisie parmi l'huile de graine de coriandre, l'huile de graine de bourrache, l'huile d'onagre, l'huile de maïs, l'huile de tournesol, l'huile de carthame, et l'huile d'algue, de préférence l'huile de graine de tournesol.

9. Composition selon l'une quelconque des revendications précédentes, comprenant un antioxydant choisi parmi le butylhydroxytoluène, l'hydroxyhydrocinnamate de pentaérythrityle tétra-di-tert-butyle (vendu sous le nom Tinogard TT), ou le thiodipropionate de dilauryle (vendu sous le nom Tinogard DA).

10. Composition selon l'une quelconque des revendications précédentes, dans laquelle l'huile insaturée est présente dans la composition en une quantité située dans la plage allant de 0,05 % en poids à 10 % en poids.

11. Composition selon l'une quelconque des revendications précédentes, comprenant 0,01 % en poids à 2 % en poids, par rapport au poids de la composition, de composé antioxydant.

12. Composition antitranspirante selon l'une quelconque des revendications précédentes, qui est délivrée sous la forme d'un bâtonnet, d'un gel, d'un dispositif à bille, ou dans un bidon aérosol.

13. Procédé pour minimiser la formation de taches ou d'une coloration jaunâtre sur une étoffe portée par un individu, comprenant les étapes (a) d'application d'une composition selon l'une quelconque des revendications précédentes sur une partie du corps, laquelle composition appliquée sur une partie du corps vient en contact avec l'étoffe quand celle-ci est portée par un individu, de préférence l'aisselle de l'individu, suivie de (b) un lavage, (c) un rinçage et (d) éventuellement un séchage de l'étoffe.

14. Utilisation de 1 % en poids à 20 % en poids de tensioactif siliconé non-ionique ayant un indice HLB inférieur à 11 dans une composition antitranspirante comprenant (i) un principe actif antitranspirant à base de métal, (ii) une huile insaturée ayant un indice d'iode d'au moins 100 et (iii) moins de 10 % en poids d'eau pour minimiser la formation de taches sur des étoffes qui viennent en contact avec la composition.

15. Utilisation de 1 % en poids à 20 % en poids de tensioactif siliconé non-ionique ayant un indice HLB inférieur à 11 dans une composition antitranspirante comprenant (i) un principe actif antitranspirant à base de métal, (ii) une huile insaturée ayant un indice d'iode d'au moins 100 et (iii) moins de 10 % en poids d'eau pour réduire la perception par le consommateur de marques blanches sur des étoffes qui viennent en contact avec la composition.
